# EUROPEAN PATENT APPLICATION

(11) **EP 2 233 471 A1**
(43) Date of publication of application: **29.09.2010**
(21) Application number: 09460007.9
(22) Date of filing: 06.02.2009
(51) Int. Cl.: C07D 215/22, A61K 31/4704, A61P 25/00

(54) **A salt of 7-{4-[4-(2,3-dichlorophenyl)-1-piperazinyl]butoxy}-3,4.dihydro-2(1h)-quinolinone with 5-sulfosalicylic acid and its preparation process**

(71) Applicant: Adamed Sp. z o.o., 05-152 Czosnów k/Warszawy (PL)
(72) Inventor: Kludkiewicz, Dominik Daniel, 03-242 Warszawa (PL); Rusiecki, Rafal, 01-891 Warszawa (PL)
(74) Representative: Witek, Rafal

(57) **Abstract**

7-{4-[4-(2,3-dichlorophenyl)-1-piperazinyl]butoxy}-3,4-dihydro-2(1H)-quinolinone salt with 5-sulfosalicylic acid and process of preparation thereof have been disclosed. On account of advantageous physico-chemical properties this salt may be applied for the manufacturing of pharmaceuticals, especially for therapeutics useful in schizophrenia treatment.

## Description

### Field of invention

The present invention relates to 7-{4-[4-(2,3-dichtorophenyl)-1-piperazinyl]butoxy}-3,4-dihydro-2(1H)-quinolinone salt with 5-sulfosalicylic acid and its process of preparation.

On account of advantageous physico-chemical properties this salt may be applied for the manufacturing of pharmaceuticals, especially for therapeutics useful in schizophrenia treatment.

7-{4-[4-(2,3-dichlorophenyl)-1-piperazinyl]butoxy}-3,4-dihydro-2(1H)-quinolinone (aripiprazole base) may be prepared by methods known from the state of art, *e*.*g*. by the method disclosed in European patent EP-B-0367141 consisting of 7-(4-bromobutoxy)-3,4-dihydro-2(1*H*)quinolinone condensation with 1-(2,3-dichlorophenyl)piperazine. The process is performed in the presence of potassium iodide and triethylamine during heating at boiling temperature.

The method of aripiprazole synthesis in water solution of potassium carbonate is disclosed in EP-B-1330249. According to this method anhydrous B form of aripiprazole may be obtained. As represented by the given below structure of formula (I):

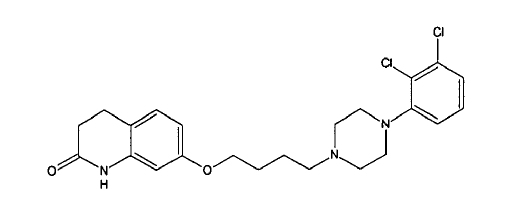

Aripiprazole is used for treatment of schizophrenia **(**EP-B-367141**)** as well as episodes of mania in type I bipolar disorder. Aripiprazole *in vitro* affinity profile toward dopamine and serotonin receptors resembles atypical antipsychotic agents. Complex mechanism of aripiprazole action is postulated as it reveals antagonistic influence on HT_{2A} receptors as well as partial agonistic influence on dopamine D2 and serotonin 5-HT_{1A} receptors.

Several aripiprazole salts are known from the prior art, *e*.*g*. European patent application EP-A-1330249 discloses hydrate of aripiprazole A form. EP-A-1797039 reveals aripiprazole p-toluenosulfonate, benzenosulfonate, citrate, salicylate and hydrobromide. Aripiprazole alkali metals salts are known from European application EP-A-367141**.** Moreover from the prior art aripiprazole hydrochloride (EP-A-1606262), pamoate (EP-A-1651219), salts with dibasic organic acids, *i*.*e*. oxalic, tartaric and succinic acids (EP-A-1907364) are known. Aripiprazole co-crystals with fumaric acid (WO 2007/092779), aripiprazole hemifumarate (WO 2008/034628) as well as its additive salts - hydroiodide, hydrobromide, oxalate, dimethylbenzenesulfonate, tetrafluoroborate, phosphate, phenylphosphate (EP-A-1686126) are also revealed. Aripiprazole oxalate, benzoate form I and II, maleate, malonate, fumarate, L-tartate, malate and citrate are disclosed in (EP-A-1837331).

Aripiprazole base as well as some of its salts reveal polymorphic properties. The pharmaceutical industry is frequently confronted with the problematic phenomenon of multiple crystal polymorphs of the same chemical entity. The presence of multiple polymorphs of the active pharmaceutical ingredient is particularly challenging with solid, oral dosage drug products ^{[1,2]}. Polymorphism is often characterized as the ability of a drug substance to exist as two or more crystalline phases that have different arrangements and /or conformations of the molecules in the crystal lattices ^{[3]}. Sometimes the properties of two solid forms of a drug are quite similar. In other cases, the physical and chemical properties can vary dramatically and have great impact on pharmacokinetics, ease of manufacturing, and dosage form stability. Properties that can differ among solid forms of a substance include color, solubility, crystal shape, water sorption and desorption properties, particle size, hardness, drying characteristics, flow and filterability, compressibility, and density. Different solid forms can have different melting points, spectral properties, and thermodynamic stability

However, there still exists a need for a form of aripiprazole which is stable, reproducible and in a suitable form for being further processed into pharmaceutical compositions showing a high content uniformity.
For a person skilled in art it is evident, that all aripiprazole salts may reveal polymorphic nature, as it was proven for aripiprazole benzoate - which polymorphic forms I and II are described in European patent application EP-A- 1837331**.**

In a drug substance, these variations in properties can lead to differences in dissolution rate, oral absorption, bioavailability, toxicology results and clinical trial results. Ultimately both safety and efficacy are impacted by properties that vary among different solid forms.

Furthermore, stability presents a special concern, since it's easy to inadvertently generate the unintentional form at any point in the development process. Because energy differences between forms are usually relatively small, form interconversion is common and can occur during routine API manufacturing operations and during drug product formulation, storage, and use⁴

Encountering a new solid form during late stages of development can delay filing. A new polymorphic form with disadvantageous pharmacological properties, appearing in drug product can cause product withdrawal. For this reason pharmaceutical manufacturers often select a drug substance polymorphic form that has the desirable characteristics that will aide in the manufacture of the drug product formulation. Hence, it becomes critical to have a robust crystallization process that consistently produces the desired polymorphic form of the bulk pharmaceutical active ingredient.

### Detailed description of the invention

In one aspect the present invention provides additive salt of 7-{4-[4-(2,3-dichlorophenyl)-1-piperazinyl]butoxyl-3,4-dihydro-2(1H)-quinolinone with 5-sulfosalicylic acid. As used herein "addition salt of 7-{4-[4-(2,3-dichlorophenyl)-1-piperazinyl]butoxy}-3,4-dihydro-2(1H)-quinolinone with 5-sulfosalicylic acid" refers to 5-sulfosalicylate salt of aripiprazole.

Chemical structure of this salt may be represented by the given below structure of formula (II):

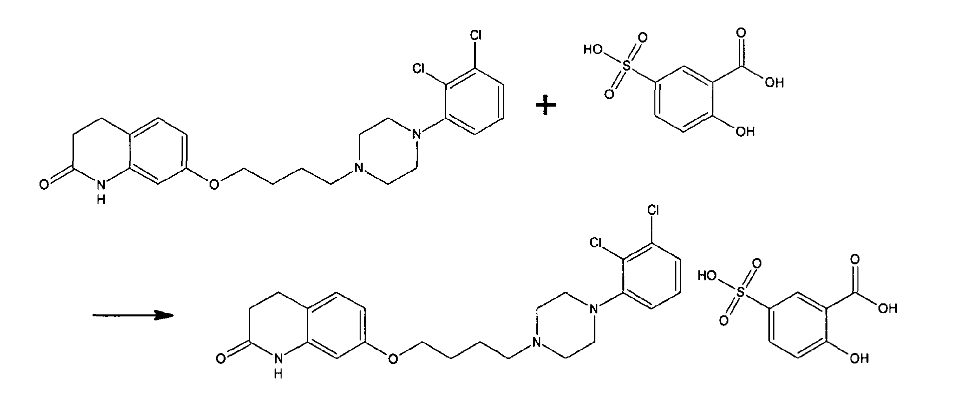

Unexpectedly, it turned out that the new aripiprazole 5-sulfosalicylate of present invention, is very stable and does not reveal polymorphic forms.

As used herein "stable aripiprazole salt" refers to lack of physico-chemical properties changes, especially such as polymorphic form interconversions, hydrates and solvates forming or increase of impurities amount tendency, in a range of tested temperature, pressure and humidity conditions.

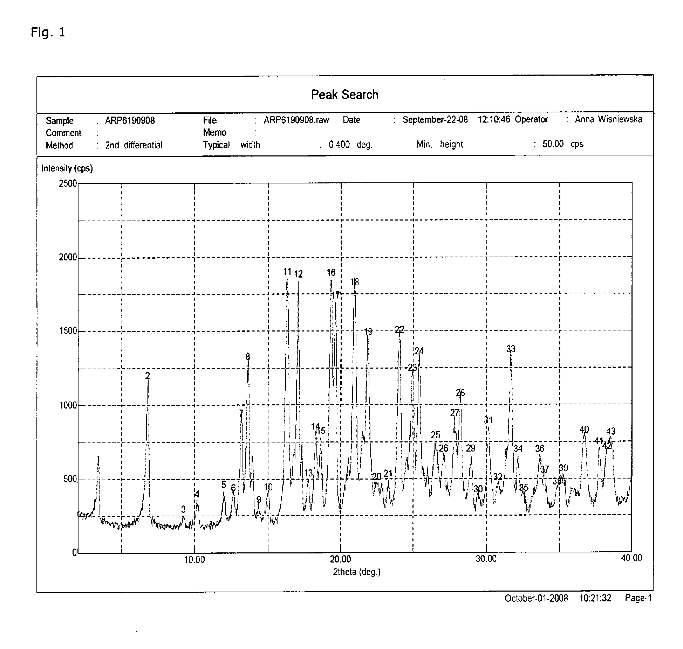

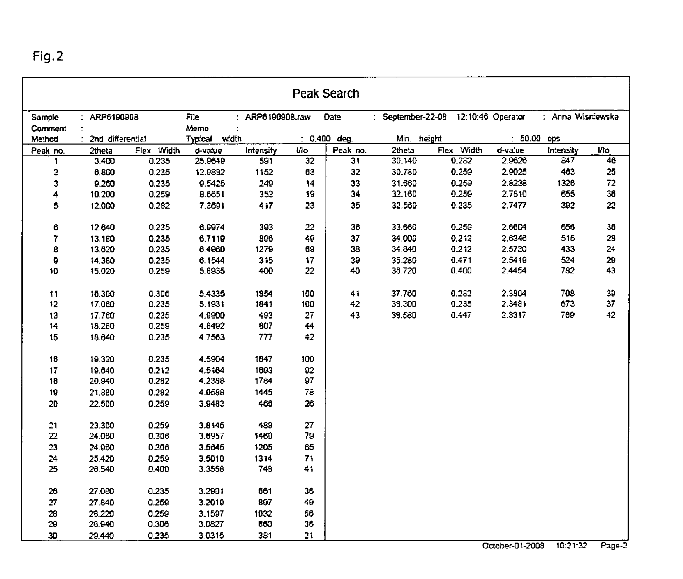

Preferably the salt of the present invention is characterized by HPLC purity above 99,8%. A further advantage of the salt of present invention is its crystalline form. Preferably crystalline salt according to the present invention reveals at least one of data selected from the group consisting of:
- Characteristic peaks present on X-ray powder diffraction pattern (XRPD) in form of relations between interplanar spacings d (Ǻ) and deflection angle 2θ (°), substantially as depicted in Fig.2
- a X-ray powder diffraction pattern (XRPD) substantially as depicted in Fig.1
- melting point in a range of 209,85 - 217,37°C determined on curve of differential scanning calorimetry as onset peak
- melting temperature in a range of 214, 50 - 217,37°C determined on curve of differential scanning calorimetry as extrapolated peak
- infrared spectrum with characteristic peaks at λ: 3185, 2993, 2908, 2834, 2713, 2623, 1683, 1627, 1582, 1477, 1314, 1291, 1243 cm-1
- X-ray powder diffraction pattern (XRPD) demonstrating characteristic peaks at 2θ, +/-0,2θ as follows:

| |
|---|
| 2θ |
| 3.4 |
| 6.8 |
| 13.2 |
| 13.6 |
| 15.0 |
| 16.3 |
| 17.1 |
| 18.3 |
| 19.3 |
| 21.9 |
| 22.5 |
| 23.3 |
| 25.0 |
| 26.6 |
| 27.8 |
| 28.2 |

In another aspect, the present invention provides process of preparation of 5-sulfosalicylate salt of aripiprazole, consist in that salt of 7-{4-[4-(2,3-dichlorophenyl)-1-piperazinyl]butoxy}-3,4-dihydro-2(1H)-quinolinone in the form of free base, suspended or dissolved in organic solvent is subjected to reaction with 5-sulfosalicylic acid, preferably with its dihydrate.

Preferably, for the salt forming reaction the molar ratio of 7-{4-[4-(2,3-dichlorophenyl)-1-piperazinyl]butoxy}-3,4-dihydro-2(1H)-quinolinone to 5-sulfosalicylic acid is about 1:1.

Suitable organic solvents are selected from the group of alcohols, nitriles, chlorohydrocarbons or mixtures thereof. Most preferably organic solvents are selected from chloroform or ethanol.

In preferable embodiment of present invention 5-sulfosalicylate salt of aripiprazole is prepared in a process comprising:
(i) mixing of equimolar quantities of 7-{4-[4-(2,3-dichlorophenyl)-1-piperazinyl]butoxy}-3,4-dihydro-2(1H)-quinolinone and 5-sulfosalicylic acid
(ii) heating the mixture at boiling temperature
(iii) product filtering off
(iv) drying of the product under reduced pressure or air-drying under atmospheric pressure

Another aspect of the present invention relates to use of the addition salt of 7-{4-[4-(2,3-dichlorophenyl)-1-piperazinyl]butoxy}-3,4-dihydro-2(1H)-quinolinone with 5-sulfosalicylic acid for preparation of pharmaceuticals useful in schizophrenia and episodes of mania in type I bipolar disorder.

### Brief description of the figures

The following figures additionally characterize the present invention
Fig.1 illustrates XRPD for aripiprazole 5-sulfosalicylate
Fig.2 illustrates XRPD characteristic peaks values presented in a form of relations between interplanar spacings d (Ǻ), deflection angle 2θ (°)

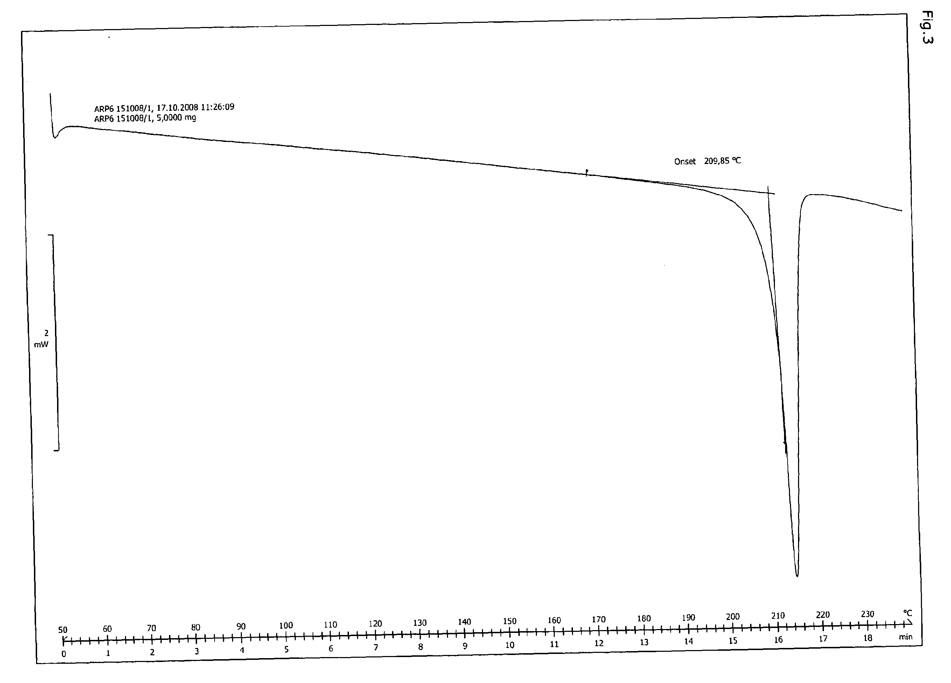

Fig.3 illustrates a DSC thermogram of aripiprazole 5-sulfosalicylate
Fig.4 illustrates HPLC for aripiprazole 5-sulfosalicylate. Nonintegrated peak observed at dead time is characteristic for 5-sulfosalicylate. ARP6 denotes aripiprazole 5-sulfosalicylate , ARP 1-4 denotes semi-products

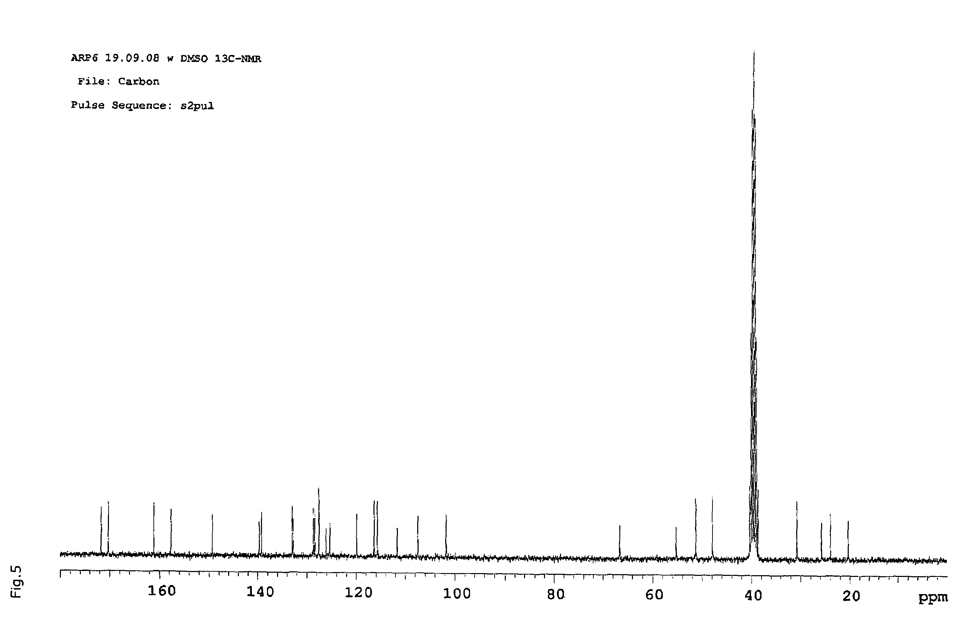

Fig.5 illustrates magnetic resonance spectrum ¹³CNMR for aripiprazole 5-sulfosalicylate

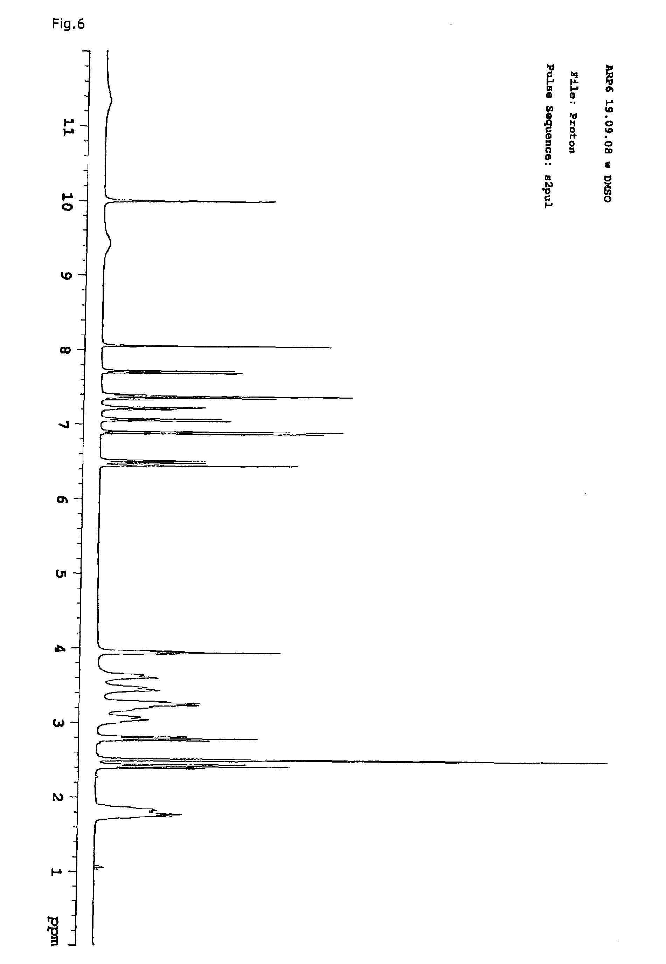

Fig.6 illustrates magnetic resonance spectrum ¹HNMR for aripiprazole 5-sulfosalicylate

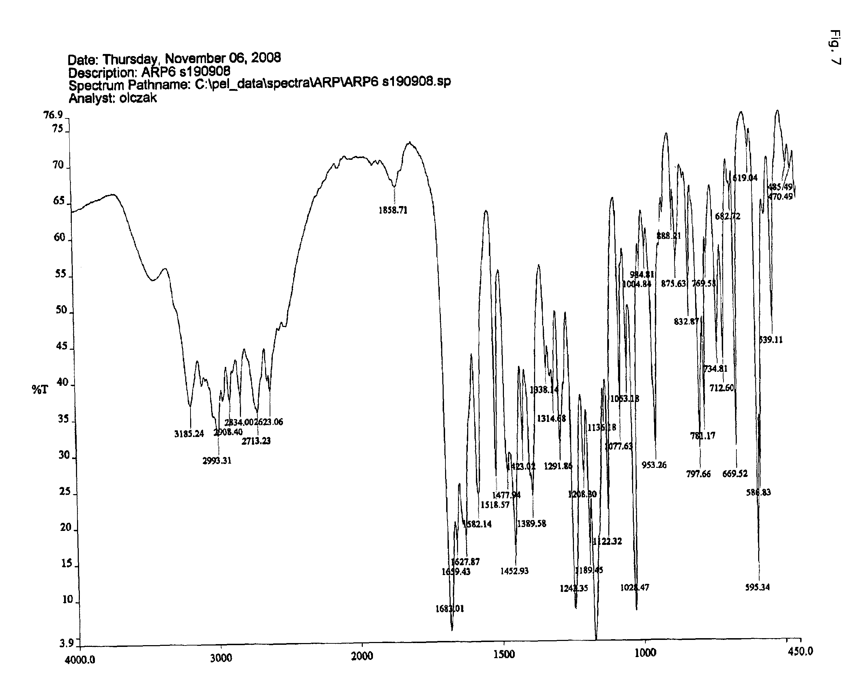

Fig.7 illustrates infrared spectrum (FT-IR, KBr) for aripiprazole 5-sulfosalicylate

For the aripiprazole 5-sulfosalicylate salt forming reaction the molar ratio of 7-{4-[4-(2,3-dichlorophenyl)-1-piperazinyl]butoxy}-3,4-dihydro-2(1H)-quinolinone to 5-sulfosalicylic acid could be about 1:1.

Suitable organic solvents are selected from the group of alcohols, nitriles, chlorohydrocarbons or mixtures thereof. Most preferably organic solvents are selected from chloroform or ethanol.

In preferable embodiment of present invention 5-sulfosalicylate salt of aripiprazole is prepared in a process comprising:
(i) mixing of equimolar quantities of 7-{4-[4-(2,3-dichlorophenyl)-1-piperazinyl]butoxy}-3,4-dihydro-2(1H)-quinolinone and 5-sulfosalicylic acid dihydrate in organic solvent or mixture of organic solvents or in a mixture of organic solvent with water
(ii) heating the mixture at reflux
(iii) filtering off the obtained precipitate while hot
(iv) drying of the product under reduced pressure or under normal pressure in laboratory dryer

Aripiprazole 5-sulfosalicylate of the present invention is obtained with yield exceeding 91% - for the reaction performed in chloroform. The salt obtained according to above presented process is characterized by very high chemical purity. HPLC purity of aripiprazole 5-sulfosalicylate usually exceeds 99,8% - without additional steps of purification.

In the most preferable embodiment of present invention aripiprazole 5-sulfosalicylate is prepared in a process comprising
(i) mixing of equimolar quantities of 7-{4-[4-(2,3-dichlorophenyl)-1-piperazinyl]butoxy}-3,4-dihydro-2(1H)-quinolinone and 5-sulfosalicylic acid dihydrate in organic solvent or mixture of organic solvents or in a mixture of organic solvent with water
(ii) heating the mixture at reflux
(iii) slow cooling down of the reaction mixture and subsequent filtering off the obtained precipitate
(iv) air-drying of the product

Aripiprazole 5-sulfosalicylate prepared according to the above presented description is obtained with yield exceeding 88% - for reaction performed in 99.8% ethanol. The salt prepared according to the above presented description is characterized by very high chemical purity. HPLC purity of aripiprazole 5-sulfosalicylate usually reaches 99,8% - without additional steps of purification.

The above described methods of aripiprazole 5-sulfosalicylate preparation may be applied for semi- production as well as production scale.

Aripiprazole 5-sulfosalicylate prepared according to the present invention is chemically stable. Its storage stability was estimated in stress conditions. Test analysis performed after 6 months storage period revealed neither additional impurities nor water content increase.

The salt of present invention contains aripiprazole base and 5-sulfosalicylic acid in molar ratio 1:1, what was confirmed by acid-base titration method as well as by ¹H-NMR and ³C-NMR

Acid-base titration method was performed using automatic titrator (Metrohm) (module 835 Titrando + 801 Stirrer) with pH-metric electrode.

Proton and carbon magnetic resonance spectra were recorded on Varian VNMRS 300 spectrophotometer at 27°C in DMSO-d6

NMR (DMSO-d6) analysis revealed characteristic signals, as follows:
**¹HNMR**: δ(ppm): 1,75-1,84 (m, 4H), 2,41 (t, 2H), 2,79 (t, 2H), 3,00-3,08 (m, 2H), 3,21-3,29 (m, 4H), 3,44-3,48 (m, 2H), 3,60-3,64 (m, 2H), 3,49 (t, 2H), 6,44 (d, 1H), 6,49 (dd, 1H), 6,89 (d, 1H), 7,06 (d, 1H), 7,21 (dd, 1H), 7,33-7,40 (m, 2H), 7,71 (dd, 1H), 8,05 (d, 1H), 9,42 (s, 1H), 10,00 (s, 1H), 11,33 (s, 1H).
**¹³CNMR:** δ(ppm): 20,34; 23,97; 25,76; 30,73; 47,91; 51,31; 55,29; 66,60; 101,74; 107,45; 111,62; 115,68; 116,37; 119,88; 125,37; 126,08; 127,52; 128,39; 128,63; 132,73; 132,94; 139,21; 139,66; 149,38; 157,66; 161,08; 170,27; 171,69.

Crystalline aripiprazole 5-sulfosalicylate is characterized by X-ray powder pattern (XRPD) presented in a form of relations between interplanar spacings d (Ǻ), deflection angle 2θ (°) and relative intensities in attitude to the most intensive diffraction line, I/I₀ (%), as depicted in Fig 1 and Fig 2.

XRPD were recorded on a *MiniFlex* (Rigaku) X-ray powder diffractometer with copper radiation (CuKα) and Kβ filtering, using acquisition condition:
θ /2Θ scanning was performed in a range from 2° to 40°, at ccanning rate (continuous) 1°/min and stepsize: every 0.02° Θ. Detection was performed on NaI scintillation counter. Obtained diffraction patterns are presented in Fig. 1 and Fig.2

Residual solvents content was characterized by gas chromatography method (GC 3800 Varian with headspace module and FID)

Chemical purity of the salt obtained according to the present invention was characterized by HPLC method (Alliance 2695 (Waters), UV detector) using conditions as presented in Table 1.

**Table 1. HPLC conditions applied for chemical purity determination of aripiprazole 5-sulfosalicylate**

| | |
|---|---|
| column | C-18, 150 x 4,6 mm; 3 µm (ACE or equivalent) |
| Column temperature | 25 °C |
| Autosampler temperature | RT (20-25 °C) |
| Detection wave length | 218 nm |
| Injection volume | 10 µl |
| Sample concentration | 0.4 mg/ml (ACN:H₂O 1:1) |
| Mobile phase | A - 0.10 % TFA in water, B - ACN |
| Linear gradient | As depicted in Table 2 . |

**Table 2: HPLC gradient parameters**

| | time | flow | %A | %B | Gradient curve |
|---|---|---|---|---|---|
| | [min] | [ml/min] | | | |
| 1 | 0 | 1.0 | 72 | 28 | - |
| 2 | 15 | 1.0 | 55 | 45 | 6 (linear) |
| 3 | 19 | 1.0 | 55 | 45 | 6 (linear) |
| 4 | 21 | 1.0 | 72 | 28 | 6 (linear) |
| 5 | 24 | 1.0 | 72 | 28 | 6 (linear) |

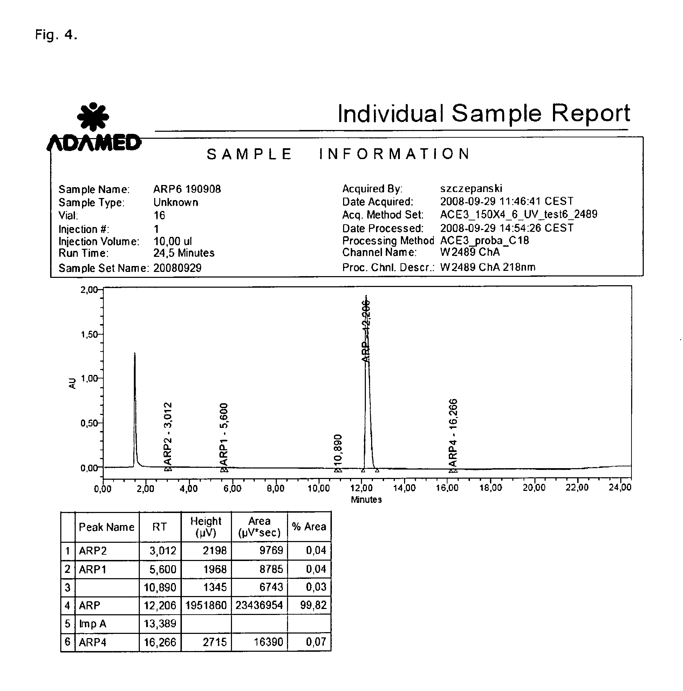

HPLC purity of obtained product was estimated as 99,82%. Results of analysis are presented on Fig.4

Water content was determined below 0.5 % (in a range from 0.2 to 0.4%).

Aripiprazole 5-sulfosalicylate stability was characterized in stress conditions (40°C; 75% RH). Comparing with starting material there were no changes observed in aripiprazole 5-sulfosalicylate sample after 6 months storage what was confirmed by DSC, HPLC, XRPD, water content analysis, elemental analysis as well as by macroscopic appearance analysis.

Another aspect of the present invention relates to the use of aripiprazole 5-sulfosalicylate, especially its crystalline form, to manufacture of therapeutics useful in treatment of schizophrenia and episodes of mania in type I bipolar disorder. Aripiprazole salt applied for treatment may reveal above presented characteristic and/or be manufactured according to above described process.
In therapeutic applications aripiprazole salt with 5-sulfosalicylic acid may be formulated in a form of pharmaceutical compositions comprising therapeutically effective amount of active substance with at least one pharmaceutically acceptable carrier and/or dilutent.
Suitable carriers for liquid form compositions may be selected from: water, oils, glycols, alcohols. For solid forms compositions suitable carriers may comprise: starch, microcrystalline cellulose, lactose or other sugars.

Pharmaceutical compositions containing aripiprazole 5-sulfosalicylate according to the invention as active ingredient in admixture with one or more carriers or auxiliary substance(s) conventionally applied in the pharmaceutical industry such as: buffers, sweetening agents, flavours and others excipients like disintegrants, lubricants, glidants, binders, disintegrants, surface-active ingredients or dispersing agents, preservatives and other supplements - *e*.*g*. ensuring isotonicity with human blood.

Pharmaceutical composition comprising the salt of present invention may be in form of powder, wafer, tablet or capsule, fast-dissolving tablet, granulate for preparing solutions, oral solution, oral suspension in water or non-water solutions, emulsions and solutions for injection.

The solid, semisolid or liquid pharmaceutical compositions according to the invention preferably inform of single dosage unit, may be produced by methods conventionally-applied in the pharmaceutical industry. Tablets comprising aripiprazole 5-sulfosalicylate may optionally be coated using one of the standard methods based on water or organic phase.

Aripiprazole 5-sulfosalicylate according to the present invention acting as D₂ dopamine and 5-HT_{IA} serotonin receptors partial agonist as well as 5-HT_{2A} receptors antagonists can be applied as antipsychotic agent, especially in schizophrenia treatment.

Active substance dose selection and dosage scheme may be determined by a person skilled in art basing on treatment and prevention algorithms appropriately for schizophrenic disorders.

Daily dosage may be administered to the patient as monotherapy or in combination with other therapeuticals.

Pharmaceutical compositions comprising aripiprazole 5-sulfosalicylate may be administered to the patient in combination with other therapeuticals, such as neuroleptics (particularly haloperidol), atypical antipsychotic agents (particularly clozapine), antidepressants (particularly mirtazapine), H2 histamine agonists (particularly famotidine), compounds containing lithium, anticonvulsants (particularly valproate), antitussives agents (particularly dextrometorphan), antithrombotics (particularly warfarine) , proton pump inhibitors (particularly omeprazol), benzodiazepines (particularly lorazepam), antipsychotics (particularly olanzapine).

Such substances may be administered to the patient in a form of one combined pharmaceutical preparation with fixed dosage regimen or one after the other in order and time intervals established by a specialist.

This invention will be further described by the following Examples which should not be construed as limiting the scope of the invention:

### Example 1. physico-chemical characteristic of aripiprazole salt of present invention

Proton and carbon magnetic resonance spectra were recorded on Varian VNMRS 300 spectrophotometer at 27°C in DMSO-d6

Residual solvents content was characterized by gas chromatography method (GC 3800 Varian with headspace module (Teledyne Tekmar) and FID)

The infrared absorption spectra (FT-IR) in a range 4000 - 400 cm ⁻¹ were measured on Thermo Nicolet spectrophotometer with Fourier transform (SpectroLab). Sample was prepared as KBr pressed tablets.

XRPD were recorded on a *MiniFlex* (Rigaku) X-ray powder diffractometer with copper radiation (CuKα) and Kβ filtering, using acquisition condition:
θ /2Θ scanning was performed in a range from 2° to 40°, at ccanning rate (continuous) 1°/min and stepsize: every 0.02° Θ. Detection was performed on NaI scintillation counter.

Melting point was measured using differential scanning calorimetry on Mettler Toledo DSC 821e.

Aripiprazole base used as starting material for below described experiments was obtained according to the method described in aripiprazole product patent EP-B-367141.

### Example 2. crystallization of aripiprazole 5-sulfosalicylate from 99.8% ethanol

5g of aripiprazole base and 2,8 g of 5-sulfosalicylic acid dihydrate dissolved in 80 ml of 99,8% ethanol were heated at reflux for 2h. Very thick, white sediment which precipitates during boiling was collected by filtration while hot and dried at 90°C under normal pressure in laboratory drier Alpina S-40 to give aripiprazole 5-sulfosalicylate in 88% yield (6,56g, white powder).
DSC onset: 214,0°C
DSC extrapolated: 216,51 °C

| Residual solvents. Component summary for amount | | | | | |
|---|---|---|---|---|---|
| | sample | Sample weight | Methanol ppm | Ethanol ppm | Acetonitrile ppm |
| 1 | ARP6 150108_2 p1 | 0.20158 | 45 | 386 | 2 |
| 2 | ARP6 150108_2 p2 | 0.20113 | | 315 | |
| Mean | | | 45 | 351 | 2 |
| Std dev. | | | | 50.4 | |
| % RSD | | | | 14.4 | |

**¹HNMR:** δ: 1,75-1,84 (m, 4H), 2,41 (t, 2H), 2,79 (t, 2H), 3,00-3,08 (m, 2H), 3,21-3,29 (m, 4H), 3,44-3,48 (m, 2H), 3,60-3,64 (m, 2H), 3,49 (t, 2H), 6,44 (d, 1H), 6,49 (dd, 1H), 6,89 (d, 1H), 7,06 (d, 1H), 7,21 (dd, 1H), 7,33-7,40 (m, 2H), 7,71 (dd, 1H), 8,05 (d, 1H), 9,42 (s, 1H), 10,00 (s, 1H), 11,33 (s, 1H).
**¹³CNMR:** δ: 20,34; 23,97; 25,76; 30,73; 47,91; 51,31; 55,29; 66,60; 101,74; 107,45; 111,62; 115,68; 116,37; 119,88; 125,37; 126,08; 127,52; 128,39; 128,63; 132,73; 132,94; 139,21; 139,66; 149,38; 157,66; 161,08; 170,27; 171,69.
**IR:** λ cm⁻¹: 3185, 2993, 2908, 2834, 2713, 2623, 1683, 1627, 1582, 1477, 1314, 1291, 1243

### Example 3. crystallization of aripiprazole 5-sulfosalicylate from chloroform

5g of aripiprazole base and 2,8 g of 5-sulfosalicylic acid dihydrate dissolved in 80 ml of chloroform were heated at reflux for 2h. The sediment which precipitates during boiling was collected by filtration while hot and dried at 90°C under normal pressure in laboratory drier Alpina S-40 to give aripiprazole 5-sulfosalicylate in 91% yield (6,73g, white powder).
DSC onset: 213,31°C
DSC extrapolated: 217,50 °C

| Residual solvents. Component summary for amount | | | |
|---|---|---|---|
| | sample | Sample weight | chloroform ppm |
| 1 | ARP6 151008_1 p1 | 0.20628 | 964 |
| 2 | ARP6 151005_1 p2 | 0.18264 | 1148 |
| Mean | | | 1056 |
| Std dev. | | | 130.2 |
| % RSD | | | 12.3 |

### Example 4. crystallization of aripiprazole 5-sulfosalicylate from chloroform/ methanol mixture (1:1 v/v)

5g of aripiprazole base dissolved in 40 mL of chloroform was mixed with and 2,8 g of 5-sulfosalicylic acid dihydrate solution in 40 ml of methanol. The obtained mixture was heated at reflux for 2h. The white sediment which precipitates slowly during boiling was allowed for crystallization at RT over night. Obtained precipitate was collected by filtration and dried at 90°C under normal pressure in laboratory drier Alpina S-40 to give aripiprazole 5-sulfosalicylate in 83% yield (6,16g, white powder).
DSC onset: 217,37°C
DSC extrapolated: 220,33 °C

| Residual solvents. Component summary for amount | | | |
|---|---|---|---|
| | sample | Sample weight | chloroform ppm |
| 1 | ARP6 150108_3 p1 | 0.20090 | 64 |
| 2 | ARP6 150108_3 p2 | 0.20063 | 62 |
| Mean | | | 63 |
| Std dev. | | | 1.5 |
| % RSD | | | 2.4 |

### Example 5. crystallization of aripiprazole 5-sulfosalicylate from acetonitrile/ methanol mixture (1:1 v/v)

5g of aripiprazole base and 2,8 g of 5-sulfosalicylic acid dihydrate were dissolved in 80 mL of acetonitrile/methanol mixture. The obtained solution was heated at reflux for 0.5h. The obtained clear solution was allowed for crystallization at RT over night. Obtained precipitate was collected by filtration and dried at 90°C under normal pressure in laboratory drier Alpina S-40 to give aripiprazole 5-sulfosalicylate in 70% yield (5.21g, white powder).
DSC onset: 216,07°C
DSC extrapolated: 218,83 °C

| Residual solvents. Component summary for amount | | | |
|---|---|---|---|
| | sample | Sample weight | acetonitrile ppm |
| 1 | ARP6 151005_4 p1 | 0.18433 | 73 |
| 2 | ARP6 151005_4 p2 | 0.21436 | 59 |
| Mean | | | 66 |
| Std dev. | | | 9.9 |
| % RSD | | | 15.1 |

### Example 6. aripiprazole 5-sulfosalicylate stability.

Stability of aripiprazole 5-sulfosalicylate was measured in stress conditions (40°C; 75% RH). After 6 months storage samples in stress storage conditions revealed neither impurities level nor water content increase tendency. No changes in polymorphic form were observed.

| Analysis type | Analysis period | | | | | conclusions |
|---|---|---|---|---|---|---|
| | start | 1 month | 2 months | 3 months | 6 months | |
| XRPD | identical with diffractio n pattern of standard | identical with diffractio n pattern of standard | identical with diffractio n pattern of standard | identical with diffractio n pattern of standard | identical with diffractio n pattern of standard | No changes in polymorphic form observed |
| HPLC | 99,8% | 99,99% | 99,94% | 99,98% | 99,95% | lack of impurities increase |
| DSC "onset" peak | 214,0°C | 215,32°C | 214,57°C | 217,35°C | 214,68°C | lack of impurities increase or degradation products increase. Presumed lack of crystalline form interconversion s |
| DSC extrapolate d peak | 216,51°C | 217,50°C | 216,71°C | 221,99°C | 218,39°C | Presumed lack of impurities increase and crystalline form interconversion s |
| Water content ( Karl Fisher method) - as mean value +/- 0,02% | 0,36% | 0,23% | 0,28% | 0,28% | 0,19% | Differences in acceptable limits of method error |
| Elemental analysis (as percentage share) | %C 53,99 %H 4,65 %N 6,40 %CI 10,61 | | | | | Estimated percentage shares correspond to theoretic values %C 53,99 %H 4,64 %N 6,28 %CI 10,68. No hydrates are formed |
| macroscopi c appearance | White powder | White powder | White powder | White powder | White powder | Parameter crucial for pharmaceutical dosage form preparation |

Within the scope of tested conditions aripiprazole 5-sulfosalicylate revealed no changes in polymorphic form what was confirmed by XRPD analysis. HPLC and DSC results confirmed no impurities level increase tendency. Stable water content level results combined with elemental analysis findings allow to draw a conclusion that aripiprazole 5-sulfosalicylate do not form hydrates under tested conditions. There was as well no tendency to form solvates observed what was proven by result of elemental analysis, residual solvents tests and ¹H-NMR i ¹³C-NMR examinations, where no additional signals from solvents were observed.

### References:

1. Yu, L.; Reutzel, S. M.; Stephenson, G. Pharm. Sci. Technol. Today, 1(3), 118, 1998.
2. Giron, D. Thermochim. Acta, 248, 1, 1995.
3. H. G. Brittain (Ed.), P1-34 "Polymorphism in Pharmaceutical Solids", Marcel Dekker, Inc., New York, 1999.
4. S. R. Byrn, R. R. Pfeiffer and J. G. Stowell, "Solid-State Chemistry of Drugs", 2nd Edition, Academic Press, 1999.

## Claims

1. An additive salt of 7-{4-[4-(2,3-dichlorophenyl)-1-piperazinyl]butoxy}-3,4-dihydro-2(1H)-quinolinone with 5-sulfosalicylic acid

2. A salt according to claim 1 having chromatographic purity above 99.8% determined by HPLC

3. A salt according to claim 1 in crystalline form

4. A salt according to claim 3, **characterized by** an X-ray powder diffraction pattern (XRPD) in form of relations between interplanar spacings d (Ǻ) and deflection angle 2θ (°) substantially as depicted in Fig. 2.

5. A salt according to claim 3, **characterized by** an X-ray diffractogram substantially as depicted in Fig.1

6. A salt according to claim 3, **characterized by** melting point Tₒₙₛₑₜ in a range 209,85 - 217,37°C determined on curve of differential scanning calorimetry as "onset" peak

7. A salt according to claim 3, **characterized by** melting point Tₑₓₜᵣ in a range 214, 50 - 217,37°C determined on curve of differential scanning calorimetry as extrapolated peak

8. A salt according to claim 3, **characterized by** infrared spectrum with characteristic peaks at λ: 3185, 2993, 2908, 2834, 2713, 2623, 1683, 1627, 1582, 1477, 1314, 1291, 1243 cm⁻¹

9. A salt according to claim 3, **characterized by** at least one of data selected from the group consisting of:
a. infrared spectrum with characteristic peaks at λ: 3185, 2993, 2908, 2834, 2713, 2623, 1683, 1627, 1582, 1477, 1314, 1291, 1243 cm⁻¹
b. X-ray diffractogram demonstrating characteristic peaks at deflection angle 2θ +/- 0,2 θ
| |
|---|
| 2θ |
| 3.4 |
| 6.8 |
| 13.2 |
| 13.6 |
| 15.0 |
| 16.3 |
| 17.1 |
| 18.3 |
| 19.3 |
| 21.9 |
| 22.5 |
| 23.3 |
| 25.0 |
| 26.6 |
| 27.8 |
| 28.2 |

10. A method for preparing an additive salt of 7-{4-[4-(2,3-dichlorophenyl)-1-piperazinyl]butoxyl-3,4-dihydro-2(1H)-quinolinone with 5-sulfosalicylic acid, **characterized in that** a 7-{4-[4-(2,3-dichlorophenyl)-1-piperazinyl]butoxy}-3,4-dihydro-2(1H)-quinolinone suspended or dissolved in organic solvent or mixture of organic solvents or in a mixture of organic solvent with water is reacted with 5-sulfosalicylic acid

11. A method according to claim 10, **characterized in that** applied 5-sulfosalicylic acid to 7-{4-[4-(2,3-dichlorophenyl)-1-piperazinyl]butoxy}-3,4-dihydro-2(1H)-quinolinone molar ratio is about 1:1.

12. A method according to claim 10, **characterized in that** the organic solvent is selected from the group comprising alcohols, nitriles, chlorohydrocarbons or mixtures thereof

13. A method according to claim 12, **characterized in that** the organic solvent is chloroform.

14. A method according to claim 12, **characterized in that** the organic solvent is ethanol

15. A method according to claim 10, **characterized by** comprising: mixing of equimolar quantities of 7-{4-[4-(2,3-dichlorophenyl)-1-piperazinyl]butoxy}-3,4-dihydro-2(1H)-quinolinone and 5-sulfosalicylic acid in organic solvent, heating the mixture at reflux, product separation by filtration, drying of the product at elevated temperature under atmospheric pressure or at room temperature .

16. A method according to any claims from 10 to 15, **characterized in that** the 5-sulfosalicylic acid is in the form of dihydrate

17. Use of an additive salt of 7-{4-[4-(2,3-dichlorophenyl)-1-piperazinyl]butoxy}-3,4-dihydro-2(1H)-quinolinone with 5-sulfosalicylic acid, possibly in crystalline form, for preparation of medicaments for treatment schizophrenia or episodes of mania in type I bipolar disorders.
